# EUROPEAN PATENT APPLICATION

(11) **EP 4 628 080 A1**
(43) Date of publication of application: **08.10.2025**
(21) Application number: 23898321.7
(22) Date of filing: 29.11.2023
(51) Int. Cl.: A61K 31/437, A61K 31/43, A61K 31/7048

(54) **PHARMACEUTICAL COMPOSITION FOR PREVENTION OR TREATMENT OF DRUG-RELATED PEPTIC ULCER COMPRISING ZASTAPRAZAN OR PHARMACEUTICALLY ACCEPTABLE SALT THEREOF**

(30) Priority: 29.11.2022 KR 20220163540
(71) Applicant: Jeil Pharmaceutical Co., Ltd., Seoul 06543 (KR); Onconic Therapeutics Inc., Gangnam-gu Seoul 06236 (KR)
(72) Inventor: KIM, John, Seoul 06732 (KR); CHA, Hyun Ju, Seoul 06714 (KR); HAN, Sang Woo, Seoul 06274 (KR)
(74) Representative: Louis Pöhlau Lohrentz
(86) International application number: PCT/KR2023/019493
(87) International publication number: WO 2024/117797

(57) **Abstract**

Disclosed in the present invention is a pharmaceutical composition for the prevention or treatment of drug-related peptic ulcer, comprising zastaprazan or a pharmaceutically acceptable salt thereof.

## Description

### TECHNICAL FIELD

The present invention relates to a use of zastaprazan for the prevention, treatment, or recurrence prevention of drug-related peptic ulcer. Specifically, it relates to a pharmaceutical composition capable of preventing, treating, and preventing the recurrence of peptic ulcer induced by administration of nonsteroidal anti-inflammatory drugs (NSAIDs) by inhibiting or delaying its onset and inhibiting its recurrence.

### BACKGROUND ART

As the continuous use of nonsteroidal anti-inflammatory drugs (NSAIDs) as the primary therapeutic drug for chronic diseases increases, gastrointestinal side effects due to the drugs, such as peptic ulcer and bleeding, are often observed in frontline clinical settings. This is because, although NSAIDs have the role of relieving pain, long-term use thereof makes the gastric mucosal folds more vulnerable to ulcers and damage. In a past large-scale randomized controlled study, the incidence rate of symptomatic peptic ulcer was reported to be 2.7% to 4.5% in the NSAIDs use group, and that of major complications such as bleeding or perforation was reported to be 1.0% to 1.5%.

When peptic ulcer is left untreated, serious complications such as bleeding in the stomach or duodenum, perforation of the stomach or duodenal wall, ulcer penetration into other organs through the stomach or duodenum, and obstruction of food movement from the stomach to the duodenum may occur or recur frequently.

Ultimately, it is important to prevent and treat the onset of peptic ulcer caused by NSAIDs, and furthermore, there is an urgent need for a means to safely and effectively prevent the recurrence of peptic ulcer without side effects when taking NSAIDs after peptic ulcer is cured.

### [Prior Art Documents]

### [Patent Document]

(Patent Document 1) Korean Patent Publication No. 10-1777971

### DETAILED DESCRIPTION OF THE INVENTION

### TECHNICAL PROBLEM

The present inventors have identified the need for research and development of a pharmaceutical composition capable of safely and effectively preventing the development of peptic ulcer, treating them, and further preventing recurrence thereof without side effects despite long-term administration of nonsteroidal anti-inflammatory drugs (NSAIDs). Accordingly, an object of the present invention is to provide a pharmaceutical composition for preventing, treating, and preventing recurrence of peptic ulcer induced by long-term administration of NSAIDs in a safe, long-term, and effective manner without side effects, using zastaprazan, a pharmaceutically acceptable salt thereof, a hydrate or solvate thereof, or a mixture thereof.

### TECHNICAL SOLUTION

In order to achieve the above-described object, the present invention discloses the following means.

In one aspect, the present invention discloses a pharmaceutical composition for preventing, treating, or preventing recurrence of peptic ulcer, comprising zastaprazan, a pharmaceutically acceptable salt thereof, a hydrate or solvate thereof, or a mixture thereof, wherein the peptic ulcer is caused by administration of nonsteroidal anti-inflammatory drugs (NSAIDs).

### ADVANTAGEOUS EFFECTS

The present invention relates to a pharmaceutical composition for preventing, treating, or preventing recurrence of peptic ulcer, comprising zastaprazan, a pharmaceutically acceptable salt thereof, a hydrate or solvate thereof, or a mixture thereof. The pharmaceutical composition according to the present invention is capable of preventing the onset of peptic ulcer, treating it, and preventing recurrence thereof despite continued and repeated administration of nonsteroidal anti-inflammatory drugs (NSAIDs) over a long period of time.

The pharmaceutical composition for preventing, treating or preventing recurrence of peptic ulcer, comprising zastaprazan, a pharmaceutically acceptable salt thereof, a hydrate or solvate thereof, or a mixture thereof can exhibit sufficient efficacy because even co-administration of zastaprazan of the present invention with NSAIDs does not affect the pharmacokinetic (PK) characteristics of each drug.

### DESCRIPTION OF THE DRAWINGS

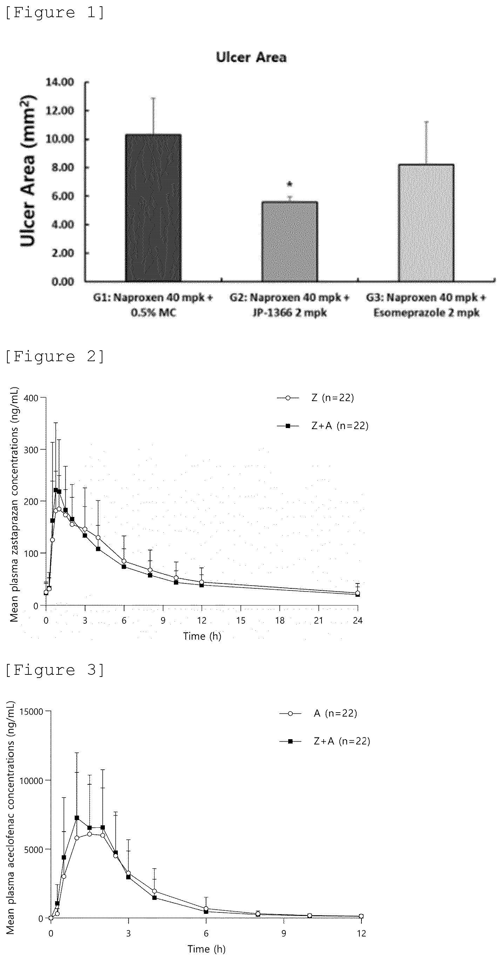

FIG. 1 shows the results of measuring the ulcer area when zastaprazan and esomeprazole were co-administered in a preclinical animal model of naproxen-induced peptic ulcer.
FIGS. 2 and 3 show whether the co-administration of zastaprazan and aceclofenac affects the pharmacokinetic (PK) profiles of the two individual drugs, illustrating the mean plasma concentration-time profiles of zastaprazan citrate (FIG. 2) and aceclofenac (FIG. 3). (In FIGS. 2 and 3, the error bars represent standard deviations.)

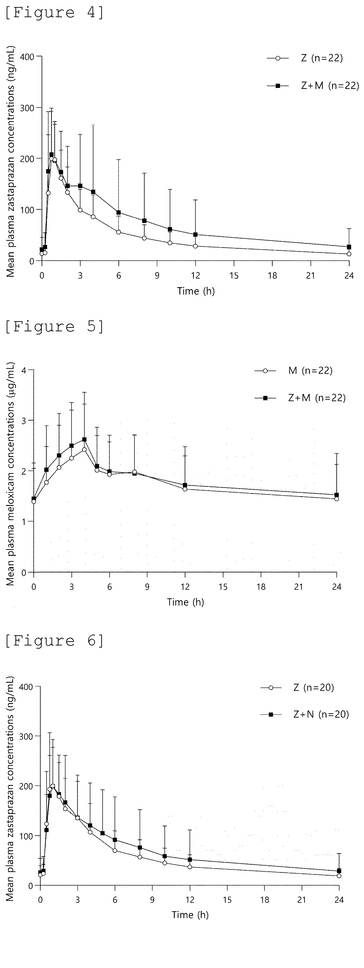

FIGS. 4 and 5 show whether the co-administration of zastaprazan and meloxicam affects the PK profiles of the two individual drugs, illustrating the mean plasma concentration-time profiles of zastaprazan citrate (FIG. 4) and meloxicam (FIG. 5). (In FIGS. 4 and 5, the error bars represent the standard deviations.)

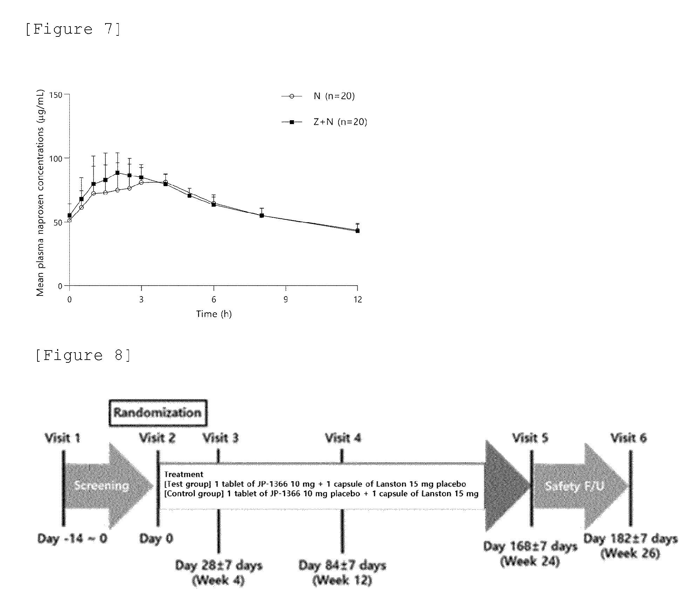

FIGS. 6 and 7 show whether the co-administration of zastaprazan and naproxen affects the PK profiles of the two individual drugs, illustrating the mean plasma concentration-time profiles of zastaprazan citrate (FIG. 6) and naproxen (FIG. 7). (In FIGS. 6 and 7, the error bars represent the standard deviations.) FIG. 8 schematically summarizes the clinical trial method in Example 3.

### BEST MODE

Hereinafter, the present invention will be described in more details.

This is explained specifically as follows. The terms used herein are selected from the most widely used general terms possible while considering the functions in the present invention, but they may vary depending on the intention of those skill in the art, precedents, the emergence of new technologies, or the like. In addition, in certain cases, there are terms arbitrarily selected by the applicant, and in this case, the meanings thereof will be described in detail in the corresponding part of the detailed description of the invention. Therefore, the terms used herein should be defined based on the meanings of the terms and the overall contents of the present invention, rather than simply the names of the terms.

Unless otherwise defined, all terms used herein, including technical and scientific terms, have the same meaning as generally understood by one of ordinary skill in the art to which the present invention pertains. Terms, such as those defined in commonly used dictionaries, should be interpreted as having a meaning that is consistent with their meaning in the context of the relevant art and are not interpreted in an idealized or overly formal sense unless clearly so defined in the present invention.

Numerical ranges are inclusive of the values defined therein. Every maximum numerical limitation given throughout the present specification comprises every lower numerical limitation, as if such lower numerical limitations were expressly written. Every minimum numerical limitation given throughout the present specification includes every higher numerical limitation, as if such higher numerical limitations were expressly written. Every numerical limitation given throughout the present specification will include every better numerical range within the broader numerical range, as if the narrower numerical limitations were expressly written.

Hereinafter, each description and embodiment disclosed in the present invention may also be applied to other descriptions and embodiments, respectively. In other words, all combinations of various elements disclosed in the present invention fall within the scope of the present invention. In addition, the scope of the present invention may not be considered as being limited by the specific description described below.

As used herein, expressions such as "comprising" are to be understood as open-ended terms implying the possibility of including other embodiments, unless specifically stated otherwise in the phrase or sentence in which the expression is included.

The term "zastaprazan" used herein may refer to zastaprazan, a pharmaceutically acceptable salt thereof, a hydrate or solvate thereof, or a mixture thereof. Therefore, "a composition including zastaprazan" as used herein may refer to a composition including zastaprazan, a pharmaceutically acceptable salt thereof, a hydrate or solvate thereof, or a mixture thereof.

Hereinafter, the present invention will be described in detail.

### Pharmaceutical composition comprising zastaprazan

The present invention discloses a pharmaceutical composition comprising zastaprazan.

Specifically, the present invention provides a pharmaceutical composition comprising zastaprazan, a pharmaceutically acceptable salt thereof, a hydrate or solvate thereof, or a mixture thereof for preventing, treating, or preventing recurrence of peptic ulcer, wherein the peptic ulcer is caused by administration of nonsteroidal anti-inflammatory drugs (NSAIDs).

The pharmaceutical composition of the present invention can prevent or treat and prevent recurrence of peptic ulcer caused by administration of NSAIDs in a safe, long-term, and effective manner without side effects for a subject who continuously takes NSAIDs for a long period of time.

As the number of elderly patients with chronic diseases such as musculoskeletal diseases or cardiovascular diseases increases, the number of people taking NSAIDs for a long period of time is also increasing. However, NSAIDs damage the stomach wall by suppressing the production of gastric mucosa and stimulating the secretion of gastric acid, and since NSAIDs are in a tablet form, most of them dissolve in the stomach and begin to be absorbed, which can physically stimulate the gastric mucosa and cause peptic ulcer when taken for a long period of time. Even when peptic ulcer is cured, peptic ulcer is likely to recur by the use of NSAIDs.

The present inventors have developed a pharmaceutical composition comprising zastaprazan, a pharmaceutically acceptable salt thereof, a hydrate or solvate thereof, or a mixture thereof. This pharmaceutical composition is capable of preventing peptic ulcer while preventing the recurrence of peptic ulcer by the use of NSAIDs.

In the present invention, zastaprazan is an example of an imidazo[1,2-a]pyridine derivative, and its chemical name is azetidin-1-yl-[8-[(2,6-dimethylbenzyl)amino]-2,3-dimethylimidazo[1,2-a]pyridin-6-yl]methanone.

In the present invention, zastaprazan is an active ingredient for preventing, treating or preventing recurrence of peptic ulcer.

In the present invention, the term "active ingredient" as used herein refers to a substance or group of substances that are expected to directly or indirectly exhibit the efficacy and effect of the pharmaceutical composition through inherent pharmacological action, and includes a main ingredient or effective ingredient.

The zastaprazan of the present invention may be present in the form of a pharmaceutically acceptable salt, and as a salt, an acid addition salt formed by a pharmaceutically acceptable free acid is useful. In the present invention, the term "pharmaceutically acceptable salt" as used herein refers to any organic or inorganic acid addition salt of the zastaprazan having a concentration that has relatively non-toxic and harmless effect on a patient, and the side effects caused by the salt do not reduce the beneficial efficacy of zastaprazan. Organic acids and inorganic acids may be used as free acids, and inorganic acids such as hydrochloric acid, phosphoric acid, sulfuric acid, nitric acid, or tartaric acid may be used, and organic acids such as methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, or hydroiodic acid may be used.

Pharmaceutically acceptable salts of the present invention comprise salts of acidic or basic groups which may be present in zastaprazan, unless otherwise indicated. For example, pharmaceutically acceptable salts may comprise sodium, calcium and potassium salts of hydroxyl groups, and other pharmaceutically acceptable salts of amino groups comprise hydrobromide, sulfate, hydrogen sulfate, phosphate, hydrogen phosphate, dihydrogen phosphate, acetate, succinate, citrate, tartrate, lactate, mandelate, methanesulfonate (mesylate) and p-toluenesulfonate (tosylate) salts, and the like, and may be prepared by methods for preparing salts known in the art.

Specifically, the pharmaceutically acceptable salt of zastaprazan may be a zastaprazan citrate salt (azetidin-1-yl{8-[(2,6-dimethylbenzyl)amino]-2,3-dimethylimidazo[1,2-a]pyridin-6-yl}methanone citrate salt), but is not limited thereto.

In the present invention, the term "hydrate" as used herein refers to a hydrate formed by binding the active ingredient, zastaprazan or a pharmaceutically acceptable salt thereof, to water by non-covalent intermolecular forces, and it contains a stoichiometric or non-stoichiometric amount of water. Specifically, the hydrate may contain water at a ratio of about 0.25 mol to about 10 mol based on 1 mol of the active ingredient, and more specifically, may contain about 0.5 mol, about 1 mol, about 1.5 mol, about 2 mol, about 3 mol, about 5 mol, or the like.

In the present invention, the term "solvate" as used herein refers to a compound formed by binding the active ingredient, zastaprazan or a pharmaceutically acceptable salt thereof, and a solvent by non-covalent intermolecular forces, and it contains a stoichiometric or non-stoichiometric amount of the solvent. Preferred solvents are volatile and non-toxic and may be administered to humans in very small amounts. Examples thereof comprise methanol, ethanol, 1-propanol, 2-propanol, 1-butanol, 2-butanol, 1-acetate, acetone, acetic acid, anisole, tetrahydrofuran, methyl acetate, ethyl acetate, propyl acetate, isopropyl acetate, isobutyl acetate, n-butyl acetate, dimethyl sulfoxide, pentane, and heptane, but the solvate of the present invention is not limited to these examples, and specifically, the solvate may contain solvent at a ratio of about 0.25 mol to about 10 mol based on 1 mol of the active ingredient, and more specifically, may contain about 0.5 mol, about 1 mol, about 1.5 mol, about 2 mol, about 3 mol, about 5 mol, or the like.

In the present invention, the term "peptic ulcer" as used herein refers to a defect in the gastric mucosa caused by an attack by gastric acid and pepsin, and histologically, a defect in the necrotic mucosa occurring below the mucosal layer is referred to as peptic ulcer. A peptic ulcer refers to an ulcer caused by an imbalance between a defensive factor that protects the mucosa and an aggressive factor that causes mucosal damage. Peptic ulcer is a general term for both gastric ulcer and duodenal ulcer and is used synonymously with gastroduodenal ulcer.

In the present invention, the term "subject" as used herein refers to a mammal, and specifically, mammals comprising humans comprise mammals such as humans, monkeys, cows, horses, dogs, cats, rabbits, rats, and mice, and more specifically, may mean humans. The subject may be a person who has developed peptic ulcer or is at risk of developing peptic ulcer due to continuous and/or long-term use of NSAIDs, or a person who has developed peptic ulcer before administering the pharmaceutical composition according to the present invention, and whose peptic ulcer has since been cured.

In the present invention, the term "cured subject" as used herein refers to a mammal, and means a subject who has been confirmed by endoscopic examination to have no ulcer, bleeding, perforation of the stomach or duodenal wall, and the like, prior to administration of the pharmaceutical composition according to the present invention after having developed peptic ulcer in the past.

In the present invention, the subject is a person who has experienced peptic ulcer at least once due to continuous and/or long-term use of NSAIDs before administering the pharmaceutical composition of the present invention, but has been cured, and the onset or complete cure of the peptic ulcer of the subject may be determined through an endoscopic examination. At this time, when bleeding or blood clots, or concentration of mucosal rugae are confirmed through an endoscopic examination in the upper gastrointestinal tract, such as the stomach or duodenum, it may be considered that a peptic ulcer has occurred. Specifically, when the results of the endoscopic examination of the upper gastrointestinal tract in the stomach or duodenum are diagnosed as an active ulcer (A1, A2) or a healing ulcer (H1, H2) according to the Sakita-Miwa classification, it may be considered that a peptic ulcer has occurred.

In the present invention, symptoms of peptic ulcer may comprise heartburn, chest tightness, pyrosis, acrid eructation, chest pain, and the like.

In the present invention, the expression "preventing recurrence of peptic ulcer" as used herein means preventing recurrence of peptic ulcer by fundamentally blocking or suppressing the onset of peptic ulcer after having experienced peptic ulcer at least once and then cured, and "preventing peptic ulcer" means delaying the onset of peptic ulcer. When the onset of peptic ulcer is delayed for an expected period of time, prevention may be considered complete.

In the present invention, the NSAIDs may be acetic acid-derived non-selective NSAIDs, enolic acid-derived non-selective NSAIDs, fenamic acid-derived non-selective NSAIDs, p-amino phenol-derived non-selective NSAIDs, propionic acid-derived non-selective NSAIDs, salicylic acid-derived non-selective NSAIDs, selective COX-2 inhibitors, or mixtures thereof.

In the present invention, the acetic acid-derived non-selective NSAIDs may be aceclofenac, acemetacin, actarit, alcofenac, amfenac, clometacin, diclofenac, etodolac, felbinac, fenclofenac, indometacin, ketorolac, metiazinic acid, mofezolac, nabumetone, naproxen, oxametacin, sulindac, zomepirac, or any combination thereof.

In the present invention, the enolic acid-derived NSAIDs may be droxicam, isoxicam, lornoxicam, meloxicam, piroxicam, tenoxicam, or any combination thereof.

In the present invention, the fenamic acid-derived NSAIDs may be flufenamic acid, mefenamic acid, meclofenamic acid, tolfenamic acid, or any combination thereof.

In the present invention, the p-amino phenol-derived NSAIDs may be paracetamol, phenacetin, or any combination thereof.

In the present invention, the propionic acid-derived NSAIDs may be alminoprofen, benoxaprofen, dexketoprofen, fenoprofen, flurbiprofen, ibuprofen, indoprofen, ketoprofen in a pharmaceutically acceptable form, loxoprofen, oxaprozin, pranoprofen, suprofen, or any combination thereof.

In the present invention, the salicylic acid-derived NSAIDs may be acetylsalicylic acid, diflunisal, salsalate, or any combination thereof.

In the present invention, the selective COX-2 inhibitors may be celecoxib, rofecoxib, valdecoxib, parecoxib, lumiracoxib, etoricoxib, firocoxib, or any combination thereof.

In the present invention, the NSAIDs may specifically comprise one or more selected from the group consisting of naproxen, aceclofenac, meloxicam, and celecoxib, but are not limited thereto.

In the present invention, the pharmaceutical composition may be administered once a day. Specifically, the zastaprazan, a pharmaceutically acceptable salt thereof, a hydrate or solvate thereof, or a mixture thereof may be administered once a day for 2 weeks or more, or 4 weeks or more, and may be administered for 24 weeks or less, or 12 weeks or less. Specifically, the pharmaceutical composition may be administered once a day for 2 to 24 weeks, 4 to 24 weeks, 4 to 12 weeks, or 2 to 12 weeks.

In the present invention, the pharmaceutical composition may comprise zastaprazan, a pharmaceutically acceptable salt thereof, a hydrate or solvate thereof, or a mixture thereof in an amount of 1 mg to 100 mg, 2 mg to 60 mg, or 3 mg to 40 mg as zastaprazan (in a free base form), specifically it may comprise zastaprazan, a pharmaceutically acceptable salt thereof, a hydrate or solvate thereof, or a mixture thereof in an amount of about 3.3, 6.6, 13.1, 26.2, or 52.4 mg as zastaprazan (in a free base form), and may comprise in an amount of 5 to 40 mg as a zastaprazan citrate salt, specifically 5 mg, 10 mg, 15 mg, 20 mg, or 40 mg as a zastaprazan citrate salt, and more specifically 10 mg, 20 mg, or 40 mg as a zastaprazan citrate salt. As such, the pharmaceutical composition of the present invention comprises a low dose of zastaprazan as described above, and when administered once a day, there is the advantage of effectively and safely preventing the recurrence of peptic ulcer or the onset of peptic ulcer caused by repeated long-term administration of NSAIDs.

In the present invention, the subject may be a person having an underlying disease such as a musculoskeletal and/or cardiovascular disease.

In the present invention, the pharmaceutical composition may comprise one or more pharmaceutical additives consisting of an excipient, a disintegrant, a binder, and a lubricant, but is not limited thereto.

In the present invention, the excipient may be one or more of microcrystalline cellulose, lactose hydrate, anhydrous lactose, sucrose, D-mannitol, starch, corn starch, or light anhydrous silicic acid, but is not limited thereto.

In the present invention, the disintegrant may comprise starch or modified starch such as sodium starch glycolate, corn starch, potato starch, or pregelatinized starch; clay such as bentonite, montmorillonite, or veegum; cellulose such as hydroxypropyl cellulose or carboxymethyl cellulose; algins such as sodium alginate or alginic acid; cross-linked celluloses such as croscarmellose sodium; gum such as guar gum or xanthan gum; cross-linked polymers such as crospovidone; effervescent agents such as sodium bicarbonate or citric acid, and the like. These may be used alone or in combination of two or more, but are not limited thereto.

In the present invention, the binder may be one or more selected from the group consisting of hydroxypropyl cellulose, hydroxypropyl methylcellulose, polyvinylpyrrolidone, copovidone, starch, microcrystalline cellulose, colloidal silicon dioxide, mannitol, lactose, polyethylene glycol, and mixtures thereof, but is not limited thereto.

In the present invention, the lubricant may be calcium stearate, glyceryl monostearate, glyceryl palmitostearate, magnesium stearate, sodium lauryl sulfate, sodium stearyl fumarate, zinc stearate, stearic acid, hardened vegetable oils, polyethylene glycol, sodium benzoate, talc, and the like. These may be used alone or in combination of two or more, but are not limited thereto.

In the present invention, the pharmaceutical composition may be a solid oral preparation, but is not limited thereto.

In the present invention, the solid oral preparation may be any one of a tablet, a film-coated tablet, a capsule, powder, a granule, a pill, a troche, an oral jelly, and an oral dissolving film, but is not limited thereto.

In particular, in the case of a film-coated tablet, a coating agent widely known in the art may be used, but is not limited thereto.

### Use for preventing recurrence or onset of peptic ulcer

The present invention provides a use of a pharmaceutical composition for preventing peptic ulcer induced by administration of NSAIDs, comprising zastaprazan, a pharmaceutically acceptable salt thereof, a hydrate or solvate thereof, or a mixture thereof.

The present invention provides a use of a pharmaceutical composition for preventing recurrence of peptic ulcer induced by administration of NSAIDs, comprising zastaprazan, a pharmaceutically acceptable salt thereof, a hydrate or solvate thereof, or a mixture thereof.

Regarding the use of the present invention, the pharmaceutical composition may comprise zastaprazan, a pharmaceutically acceptable salt thereof, a hydrate or solvate thereof, or a mixture thereof in an amount of 1 mg to 100 mg, 2 mg to 60 mg, or 3 mg to 40 mg as zastaprazan (in a free base form), specifically it may comprise zastaprazan, a pharmaceutically acceptable salt thereof, a hydrate or solvate thereof, or a mixture thereof in an amount of about 3.3, 6.6, 13.1, 26.2, or 52.4 mg as zastaprazan (in a free base form), and may comprise in an amount of 5 to 40 mg as a zastaprazan citrate salt, specifically 5 mg, 10 mg, 15 mg, 20 mg, or 40 mg as a zastaprazan citrate salt, and more specifically 10 mg, 20 mg, or 40 mg as a zastaprazan citrate salt.

As such, the pharmaceutical composition of the present invention comprises a low dose of zastaprazan as described above, and when administered once a day, there is the advantage of effectively and safely preventing the recurrence of peptic ulcer or the onset of peptic ulcer caused by repeated long-term administration of NSAIDs.

In the use for preventing recurrence or onset of peptic ulcer, the pharmaceutical composition, subject, NSAIDs, peptic ulcer, and the like of the present invention may be applied in the same way as the above-described contents as long as they do not contradict each other.

### Use for preparing a medicament for preventing recurrence or onset of peptic ulcer

The present invention provides a use of a pharmaceutical composition for preparing a medicament for preventing peptic ulcer induced by administration of NSAIDs, the pharmaceutical composition comprising zastaprazan, a pharmaceutically acceptable salt thereof, a hydrate or solvate thereof, or a mixture thereof.

The present invention provides a use for preparing a medicament for preventing recurrence of peptic ulcer induced by administration of NSAIDs, the pharmaceutical composition comprising zastaprazan, a pharmaceutically acceptable salt thereof, a hydrate or solvate thereof, or a mixture thereof.

The composition of the present invention for preparing a medicament may be mixed with an acceptable carrier or the like, and may further comprise other agents.

In the use for preparing a medicament for preventing recurrence or onset of peptic ulcer of the present invention, the pharmaceutical composition, subject, peptic ulcer, and the like may be applied in the same way as the above-described contents as long as they do not contradict each other.

### Method of preventing recurrence or onset of peptic ulcer

The present invention provides a method of preventing recurrence or onset of peptic ulcer induced by administration of NSAIDs, by administering to a subject a pharmaceutical composition comprising zastaprazan, a pharmaceutically acceptable salt thereof, a hydrate or solvate thereof, or a mixture thereof, in a pharmaceutically effective amount.

In the method, the pharmaceutical composition of the present invention, the subject, the peptic ulcer, and the like are substantially the same as those described above as long as they do not contradict each other.

In the present invention, the term "pharmaceutically effective amount" as used herein refers to an amount effective for preventing the recurrence or onset of peptic ulcer, for example, an amount of a pharmaceutical composition administered to a subject, which may comprise all amounts of the composition preventing the onset or recurrence of peptic ulcer, alleviating symptoms, inhibiting direct or indirect pathological consequences, preventing metastasis, reducing the rate of progression, mitigating or temporarily alleviating a condition, or improving the prognosis. In other words, the pharmaceutically effective amount may be interpreted as encompassing all doses at which the recurrence and/or onset of peptic ulcer may be prevented by the composition.

Specifically, in the method of preventing recurrence and/or onset of peptic ulcer according to the present invention, zastaprazan, a pharmaceutically acceptable salt thereof, a hydrate or solvate thereof, or a mixture thereof may be administered to a subject once a day in an amount of 5 mg to 100 mg as zastaprazan (in a free base form) to prevent recurrence of peptic ulcer induced by administration of NSAIDs and prevent the onset thereof. Specifically, zastaprazan, a pharmaceutically acceptable salt thereof, a hydrate or solvate thereof, or a mixture thereof, is administered to the subject in an amount of 1 mg to 100 mg, 2 mg to 60 mg, or 3 mg to 40 mg as zastaprazan (in a free base form), specifically about 3.3, 6.6, 13.1, 26.2, or 52.4 mg as zastaprazan (in a free base form), 5 to 40 mg as a zastaprazan citrate salt, preferably 5 mg, 10 mg, 15 mg, 20 mg, or 40 mg as a zastaprazan citrate salt, more preferably 10 mg, 20 mg, or 40 mg as a zastaprazan citrate salt, once a day, to effectively and safely prevent for a long time the recurrence or onset of peptic ulcer caused by the long-term continuous repeated administration of NSAIDs.

In the method of preventing recurrence or onset of peptic ulcer of the present invention, the recurrence and/or onset of peptic ulcer caused by administration of NSAIDs can be effectively and safely prevented for a long time without side effects by administration of the pharmaceutical composition to a subject, despite continuous/long-term administration of NSAIDs.

Hereinafter, the present invention will be described in more detail through manufacturing examples and examples. These manufacturing examples and examples are merely intended to explain the present invention more specifically, and the scope of the present invention is not limited by these examples.

The active ingredient used in the manufacturing examples and examples below is a zastaprazan citrate salt, which is conveniently named as zastaprazan, or code name JP-1366.

### Manufacturing Examples.

### Manufacturing Example 1. Preparation of zastaprazan citrate salt

Azetidin-1-yl{8-[(2,6-dimethylbenzyl)amino]-2,3-dimethylimidazo[1,2-a]pyridin-6-yl}methanone citrate salt was obtained according to the process described below. Specifically, azetidin-1-yl{8-[(2,6-dimethylbenzyl)amino]-2,3-dimethylimidazo[1,2-a]pyridin-6-yl}methanone was obtained according to the method described in Korean Patent Publication No. 10-1777971. The results of a nuclear magnetic resonance (NMR) analysis of the azetidin-1-yl{8-[(2,6-dimethylbenzyl)amino]-2,3-dimethylimidazo[1,2-a]pyridin-6-yl}methanone obtained above are as follows.

¹H NMR (400 MHz, CDCl₃); δ7.63(d, J=1.2 Hz, 1H), 7.13(dd, J =8.4, 6.8 Hz, 1H), 7.06-7.04(m, 2H), 6.42(d, J= 1.2 Hz, 1H), 4.86-4.84(m, 1H), 4.41-4.28(m, 4H), 4.37(d, J =4.4 Hz, 2H), 3.75-3.69(m, 1H), 2.43-2.34(m, 13H).

Next, the azetidin-1-yl{8-[(2,6-dimethylbenzyl)amino]-2,3-dimethylimidazo[1,2-a]pyridin-6-yl}methanone obtained above was mixed with an alcohol solvent (isopropyl alcohol, IPA), and the resulting mixture was stirred and then vacuum-dried at about 30 to 35 °C to obtain a dried product. About 10 g of the dried product was taken and stirred with about 167 g of acetone. To this, a solution of citric acid (about 5 g) dissolved in acetone (about 33 g) was slowly added dropwise over 60 minutes, and the resulting mixture was stirred at the same temperature for one hour. The mixture was cooled to about 20 °C to 25 °C and stirred for an additional hour. The resulting solid was filtered, washed with acetone, and dried under vacuum to obtain azetidin-1-yl{8-[(2,6-dimethylbenzyl)amino]-2,3-dimethylimidazo[1,2-a]pyridin-6-yl}methanone citrate salt. The results of an NMR analysis of the azetidin-1-yl{8-[(2,6-dimethylbenzyl)amino]-2,3-dimethylimidazo[1,2-a]pyridin-6-yl}methanone citrate salt (zastaprazan citrate salt) obtained above are as follows.

¹H NMR(400MHz, MeOD); δ 7.90(s, 1H), 7.06-7.15(m, 3H), 6.77(s, 1H), 4.50(t, J=7.2Hz, 2H), 4.45(s, 2H), 4.24(t, J=7.2Hz, 2H), 2.80(d, J=15.6Hz, 2H), 2.70(d, J=12.0, 2H), 2.39-2.44(m, 11H), 2.35(s, 3H)

### Manufacturing Example 2. Manufacture of JP-1366 tablet 10 mg (zastaprazan citrate salt 10 mg)

10 mg of azetidin-1-yl{8-[(2,6-dimethylbenzyl)amino]-2,3-dimethylimidazo[1,2-a]pyridin-6-yl}methanone citrate salt (zastaprazan citrate salt) obtained according to Manufacturing Example 1, 136.5 mg of microcrystalline cellulose, 2.3 mg of sodium stearyl fumarate, 40.0 mg of anhydrous lactose, 6.0 mg of sodium croscarmellose, and 5.2 mg of magnesium stearate were mixed to obtain a mixture. The mixture was directly pressed to obtain tablets. Next, the tablets were coated with 8.0 mg of Opadry White to prepare film-coated tablets.

The film-coated tablets manufactured as described above are referred to as 'JP-1366, 10 mg.'

### Manufacturing Example 3. Manufacture of JP-1366 tablet 20 mg (zastaprazan citrate salt 20 mg)

20 mg of azetidin-1-yl{8-[(2,6-dimethylbenzyl)amino]-2,3-dimethylimidazo[1,2-a]pyridin-6-yl}methanone citrate salt (zastaprazan citrate salt) obtained according to Manufacturing Example 1, 133.4 mg of microcrystalline cellulose, 6.4 mg of sodium stearyl fumarate, 40.0 mg of anhydrous lactose, 6.0 mg of sodium croscarmellose, and 4.2 mg of magnesium stearate were mixed to obtain a mixture. The mixture was directly pressed to obtain tablets. Next, the tablets were coated with 8.0 mg of Opadry 03B54445 Pink to prepare film-coated tablets.

The film-coated tablets manufactured as described above are referred to as 'JP-1366, 20 mg.'

### Manufacturing Example 4. Manufacture of JP-1366 capsule 20 mg (zastaprazan citrate salt 20 mg)

20 mg of azetidin-1-yl{8-[(2,6-dimethylbenzyl)amino]-2,3-dimethylimidazo[1,2-a]pyridin-6-yl}methanone citrate salt (zastaprazan citrate salt) obtained according to Manufacturing Example 1, 181.4 mg of D-mannitol, 12.4 mg of croscarmellose sodium, and 1.2 mg of magnesium stearate were mixed to obtain a mixture. The mixture was filled into hard capsules No. 1 using as a capsule base to manufacture a capsule preparation.

The capsule preparation manufactured in this way is referred to as 'JP-1366 capsule 20 mg.'

### Manufacturing Example 5. Zastaprazan 10 mg placebo

A zastaprazan 10 mg placebo was manufactured in the same manner as Manufacturing Example 2, except that the main ingredient, zastaprazan citrate salt, was not used. The film-coated tablet manufactured in this manner is referred to as 'JP-1366, 10 mg placebo.'

### Manufacturing Example 6. Manufacturing of 15 mg lansoprazole preparation (control drug)

In the case of lansoprazole preparation, 15 mg of Lanston capsule (15 mg of lansoprazole) from Jeil Pharmaceutical CO., LTD. was purchased and used. The 15 mg of purchased Lanston capsule is referred to as 'Lansoprazole, 15 mg.'

### Manufacturing Example 7. Lansoprazole 15 mg placebo

A lansoprazole 15 mg placebo was manufactured in the same manner as the Lanston capsule manufacturing method by adding a pharmaceutically acceptable excipient, except for the main ingredient lansoprazole. The capsule manufactured in this manner is referred to as 'Lansoprazole, 15 mg placebo.'

### Example 1. Evaluation of the antiulcer activity of zastaprazan in a naproxen-induced acute peptic ulcer animal model

To confirm the effect of zastaprazan in NSAID-induced peptic ulcer, the antiulcer activity of zastaprazan was evaluated in a rat gastric damage model induced by naproxen, an NSAID, and the antiulcer effect of zastaprazan was compared with that of esomeprazole.

### 1. Test animals

### Species and strains: Male Sprague-Dawley (SD) rat

Reason for selecting test system: The SD rat used in this test is an appropriate experimental animal for the efficacy evaluation test, and it was selected because abundant fundamental test data has been accumulated on the gastric mucosal damage model and thus this data may be used for the interpretation and evaluation of the test results.

### Age at acquisition: Approximately seven weeks old

Quarantine and acclimatization: Approximately seven days after acquisition (only healthy animals were provided for the test)

Individual animal identification: Individual animals were identified using the tail marking method, and an individual identification card with the test number, test group, animal ID number, and sex was attached to each housing box.
Age at test substance administration: Approximately eight weeks old
Number of animals ordered: 12
Number of animals used: 12

### 2. Housing conditions

Housing room: Clean animal room (specific pathogen free)
Ventilation frequency: At least 10 times/hour
Temperature and humidity range: 22 ± 4 °C, 50% ± 20% (RH)
Lighting time and intensity: 12 hours of lighting (lighting: 08:00 to 20:00), intensity: 150 to 300 Lux
Housing box: Polysulfonate material housing box
Number of animals per housing box: 2
Housing management: Autoclaved (121 °C, 20 minutes) bedding (BETA CHIP^{®}, Northeastern products Co., USA) was placed on the bottom of the housing box to dispose of feces and urine, and the bedding was replaced with new one twice a week.
Type and name of feed: Irradiated solid feed for rodents (PMI LabDiet^{®} 5053, USA)
Feed supply: *Ad libitum* using a feeder
Drinking water type: Tap water sterilized with a UV water sterilizer and high-pressure steam after reverse osmosis
Drinking water supply: The drinking water was replaced with new water once or twice a week, and a 500 mL rat-specific water bottle was used. The water bottle was washed and sterilized before use, and purified and UV-sterilized water was used as drinking water.

### 3. Test method

To induce gastric damage, experimental animals are orally administered naproxen at a dose of 40 mg/kg once a day for five days. The amount of liquid administered was 5 ml/kg, and four hours after the last naproxen administration, the stomach was removed by laparotomy after anesthesia.

Specifically, naproxen 40 mg/kg was orally administered once a day for five days to non-fasting SD male rats, and the stomach was removed four hours after the last naproxen administration.

Group composition and administration volume dose setting: The test group composition and administration dose are shown below.

| Group | Gastric injury model | Control / Test article | | Dose (mg/kg) | Injection volume (mℓ/kg) | Number of rats |
|---|---|---|---|---|---|---|
| G1 | Naproxen 40 mg/kg | Vehicle control | Vehicle | - | 5 | 4 |
| G2 | | Test article | JP-1366 | 2 | 5 | 4 |
| G3 | | Positive control | Esomeprazole | 2 | 5 | 4 |
| Total | | | | | | 12 |

Administration site and administration method: The test substance (zastaprazan, JP-1366) and the control substance (esomeprazole) were administered orally in combination with naproxen administration. The dose for each individual was converted based on the body weight before administration, and is administered orally in a dosage of 5 ml per kg of body weight. Administration of test substance and control substance and naproxen are performed at 30-minute intervals.

Administration frequency and administration period: Once a day for five days (a total of 5 times)

### 4. Observation and test items

### A. Observation of general symptoms

Before and after administration of the test substance, each individual was observed for general symptoms and the occurrence of dead/moribund animals. Records were maintained only for individual animals exhibiting specific symptoms (no records were made for healthy individuals).

### B. Weight Measurement

The body weight of each individual animal was measured upon acquisition, separation of groups, and before administration of the test substance. The same scale was used to minimize errors in each measurement.

### C. Autopsy and measurement of gastric damage area

After the test, the gastric damage by the test substance was evaluated using the ulcer index. Four hours after the last naproxen administration, the animal was anesthetized and bled, and the stomach was removed. The removed stomach was incised along the greater curvature, the contents are removed, and the incised stomach was washed cleanly with saline solution, spread out well with a cotton swab, fixed with pins, and then photographed. The gastric damage area and the total stomach area were measured from the photographed stomach using an image program (Leica Application Suite V4), and the ulcer index was calculated as Ulcer index (%) = Damage area (mm²) / Total area (mm²) x 100.

### D. Histopathological examination

The removed gastric tissue was fixed in a 10% neutral buffered formalin solution. The fixed tissue was trimmed to a certain thickness and then paraffin-embedded through general tissue processing to produce 4 to 5 µm thick tissue sections, and then the general staining method, Hematoxylin & Eosin staining, was performed. Hematoxylin & Eosin staining (H&E stain) slides were prepared and histopathological findings were obtained by requesting an external institution.

### 5. Statistical processing

The experimental results were statistically analyzed through Mann-Whitney analysis using a commercial statistical processing program (IBM SPSS Statistics version 25.0).

### 6. Results

Referring to FIG. 1, the group treated with the test substance, zastaprazan (JP-1366), exhibited a rapid decrease in the ulcer area in the naproxen-induced peptic ulcer model, confirming that the test substance, zastaprazan (JP-1366), showed excellent antiulcer activity.

In addition, it was confirmed that the antiulcer efficacy of the test substance, zastaprazan (JP-1366), was superior to that of the control substance, esomeprazole.

Therefore, it was confirmed that the test substance, zastaprazan (JP-1366), may prevent NSAID-induced peptic ulcer.

In addition, in Example 1, the anti-ulcer effect experiment was conducted when an NSAID was administered to the subjects in a non-fasting state, the same as when administered to actual humans. Therefore, it can be seen that the efficacy in preventing peptic ulcer caused by administration of NSAIDs was confirmed also in humans.

### Example 2. Evaluation of pharmacodynamic (PK) interaction and safety of zastaprazan with aceclofenac, meloxicam, or naproxen

Zastaprazan is a potassium-competitive gastric acid secretion inhibitor that reversibly inhibits gastric H⁺ or K⁺-adenosine triphosphatase and has a faster onset of action than proton pump inhibitors. Zastaprazan may be used not only for acid-related gastric diseases but also for preventing gastric damage caused by NSAIDs. To confirm this, the PK interaction and safety of zastaprazan with aceclofenac, meloxicam, or naproxen were clinically evaluated in healthy subjects.
- PART 1: PK interaction between zastaprazan and aceclofenac
- PART 2: PK interaction between zastaprazan and meloxicam
- PART 3: PK interaction between zastaprazan and naproxen

### 1. Test drugs

### A. Test drug 1 - Zastaprazan 40 mg

JP-1366, 20 mg 2 capsules (zastaprazan citrate salt 40 mg)

### B. Test drug 2 - Aceclofenac 100 mg

Aertal tablet (aceclofenac 100 mg)

### C. Test drug 3 - Meloxicam 15 mg

Mobic capsule 15 mg (meloxicam 15 mg)

### D. Test drug 4 - Naproxen 500 mg

Naxen-F tablet (naproxen 500 mg)

### 2. Test method

### <PART 1>

Subjects who volunteered for PART 1 were assigned a subject number on the day before the first clinical trial drug administration (-1d) of Period 1, and then administered one aceclofenac tablet on 1d of Period 1, administered two JP-1366 capsules on 4d to 8d of Period 2, and co-administered two JP-1366 capsules and one aceclofenac tablet on 9d of Period 3. Blood samples were collected from each subject at a designated time for the PK evaluation of the clinical trial drug, and all subjects underwent safety evaluation according to the designated schedule.

All subjects who completed the clinical trial underwent a post-study visit on 14d to 20d, including vital sign measurement, physical examination, adverse reaction check, confirmation of prior/concurrent medication, clinical laboratory tests, and electrocardiography.

### <PART 2>

Subjects who volunteered for PART 2 were assigned a subject number on the day before the first clinical trial drug administration (-1d) of Period 1, and then administered two JP-1366 capsules on 1d to 5d of Period 1, administered one meloxicam capsule on 15d to 19d of Period 2, and co-administered two JP-1366 capsules and one meloxicam capsule on 22d to 26d of Period 3. Blood samples were collected from each subject at a designated time for the PK evaluation of the clinical trial drug, and all subjects underwent safety evaluation according to the designated schedule.

All subjects who completed the clinical trial underwent a post-study visit on 31d to 37d, including vital sign measurement, physical examination, adverse reaction check, confirmation of prior/concurrent medication, clinical laboratory tests, and electrocardiography.

### <PART 3>

Subjects who volunteered for PART 3 were assigned a subject number on the day before the first clinical trial drug administration (-1d) of Period 1, and then administered two JP-1366 capsules on 1d to 5d of Period 1, administered one naproxen tablet on 15d to 19d of Period 2, and co-administered two JP-1366 capsules and one naproxen tablet on 22d to 26d of Period 3. Blood samples were collected from each subject at a designated time for the PK evaluation of the clinical trial drug, and all subjects underwent safety evaluation according to the designated schedule.

All subjects who completed the clinical trial underwent a post-study visit on 31d to 37d, including vital sign measurement, physical examination, adverse reaction check, confirmation of prior/concurrent medication, clinical laboratory tests, and electrocardiography.

### 3. PK analysis

Blood samples were collected at designated times for the PK evaluation of zastaprazan (JP-1366).

### A. PART 1 - 22 subjects

### (1) Aceclofenac 100 mg (A)

: 1d 0h (pre-dose), 0.25, 0.5, 1, 1.5, 2, 2.5, 3, 4, 6, 8, 10, 12h post-dose

### (2) Zastaprazan 40 mg (Z)

: 4d 0h, 6d 0h, 7d 0h, 8d 0h (pre-dose), 0.25, 0.5, 0.75, 1, 1.5, 2, 3, 4, 6, 8, 10, 12, 24h (9d 0h) post-dose

### (3) Zastaprazan 40 mg and aceclofenac 100 mg (Z+A)

: 9d 0h (pre-dose), 0.25, 0.5, 0.75, 1, 1.5, 2, 2.5, 3, 4, 6, 8, 10, 12, 24h (10d 0h) (post-dose)

### B. PART 2 - 22 subjects

### (1) Zastaprazan 40 mg (Z)

: 1d 0h, 3d 0h, 4d 0h, 5d 0h (pre-dose), 0.25, 0.5, 0.75, 1, 1.5, 2, 3, 4, 6, 8, 10, 12, 24h (6d 0h) (post-dose), 15d 0h (pre-dose)

### (2) Meloxicam 15 mg (M)

: 19d 0h (pre-dose), 1, 2, 3, 4, 5, 6, 8, 12, 24h (20d 0h) post-dose

### (3) Zastaprazan 40 mg and meloxicam 15 mg (Z+M)

: 22d 0h, 24d 0h, 25d 0h, 26d 0h (pre-dose), 0.25, 0.5, 0.75, 1, 1.5, 2, 3, 4, 5, 6, 8, 10, 12, 24h (27d 0h) post-dose

### C. PART 3 - 22 subjects

### (1) Zastaprazan 40 mg (Z)

: 1d 0h, 3d 0h, 4d 0h, 5d 0h (pre-dose), 0.25, 0.5, 0.75, 1, 1.5, 2, 3, 4, 6, 8, 10, 12, 24h (6d 0h) (post-dose), 15d 0h (pre-dose)

### (2) Naproxen 500 mg (N)

: 19d 0h (pre-dose), 0.5, 1, 1.5, 2, 2.5, 3, 4, 6, 8, 12h post-dose

### (3) Zastaprazan 40 mg and naproxen 500 mg (Z+N)

: 22d 0h, 24d 0h, 25d 0h, 26d 0h (pre-dose), 0.25, 0.5, 0.75, 1, 1.5, 2, 2.5, 3, 4, 5, 6, 8, 10, 12, 24h (27d 0h) post-dose

### 4. Results

### A. PART 1 - See Table 1 and FIGS. 2 and 3

The geometric mean ratio (GMR) [90% confidence interval, CI] of zastaprazan C_{max,SS} and AUC_{T,SS} for Z+A (zastaprazan 40 mg and aceclofenac 100 mg) to Z (zastaprazan 40 mg) was 1.0868 (0.9554 to 1.2362) and 0.9490 (0.9096 to 0.9902), respectively.

The GMR [90% CI] of aceclofenac C_{max,SS} and AUC_{T,SS} for Z+A (zastaprazan 40 mg and aceclofenac 100 mg) to A (aceclofenac 100 mg) was 1.1895 (1.0429 to 1.3568) and 1.0295 (0.9803 to 1.0812), respectively.

**[Table 1]**

| **Pharmacokinetic parameters** | **Zastaprazan** | | **Aceclofenac** | |
|---|---|---|---|---|
| | **Z+A (n=22)** | **Z (n=22)** | **Z+A (n=22)** | **A (n=22)** |
| C_{max,ss} (ng/mL) | 275.69±118.05 | 241.56±84.02 | 11769.57±2562.91 | 10095.16±3156.58 |
| AUC_{T,SS} (ng•h/mL) | 1439.39±626.62 | 1540.52±724.22 | 20825.7±3843.73 | 20418.26±4868.14 |

(Table 1 above shows the PK parameters of zastaprazan (Z) and aceclofenac (A), and the data is expressed as mean ± standard deviation.)

### B. PART 2 - See Table 2 and FIGS. 4 and 5

The GMR [90% CI] of zastaprazan C_{max,SS} and AUC_{T,SS} for Z+M (zastaprazan 40 mg and meloxicam 15 mg) to Z (zastaprazan 40 mg) was 1.0926 (0.9387 to 1.2717) and 1.3332 (1.1494 to 1.5463), respectively.

The GMR [90% CI] of meloxicam C_{max,SS} and AUC_{T,SS} for Z+M (zastaprazan 40 mg and Meloxicam 15 mg) to M (meloxicam 15 mg) was 1.0756 (0.9916 to 1.1668) and 1.0477 (0.9976 to 1.1002), respectively.

**[Table 2]**

| **Pharmacokinetic parameters** | **Tastaprazan** | | **Meloxicam** | |
|---|---|---|---|---|
| | **Z+M (n=22)** | **Z (n=22)** | **Z+M (n=22)** | **M (n=22)** |
| C_{max,ss} (ng/mL) | 268.19±118.17 | 237.21±72.79 | 2.64±0.92 | 2.48±0.88 |
| AUC_{T,SS} (ng•h/mL) | 1713.4711641.56 | 1120.01±534.42 | 44.05±18.81 | 41.82±16.42 |

(Table 2 above shows the PK parameters of zastaprazan (Z) and meloxicam (M), and the data is expressed as mean ± standard deviation.)

### C. PART 3 - See Table 3 and FIGS. 6 and 7

The GMR [90% CI] of zastaprazan C_{max,SS} and AUC_{T,SS} for Z+N (zastaprazan 40 mg and naproxen 500 mg) to Z (zastaprazan 40 mg) was 0.9148 (0.7956 to 1.0518) and 1.1089 (0.9835 to 1.2503), respectively.

The GMR [90% CI] for naproxen C_{max,SS} and AUC_{T,SS} for Z+N (zastaprazan 40 mg and naproxen 500 mg) to N (naproxen 500 mg) was 1.0491 (1.0088 to 1.0910) and 1.0155 (0.9900 to 1.0417), respectively.

**[Table 3]**

| **Pharmacokinetic parameters** | **Zastaprazon** | | **Naproxen** | |
|---|---|---|---|---|
| | **Z+N (n=22)** | **Z (n=22)** | **Z+N (n=22)** | **N (n=22)** |
| C_{max,ss} (ng/ml) | 242.34±95.02 | 263.74±88.53 | 97.40±11.49 | 92.53±8.19 |
| AUC_{T,SS} (ng•h/mL) | 1678.95±1380.41 | 1384.59±694.99 | 769.87±76.01 | 757.28±66.62 |

(Table 3 above shows the PK parameters of zastaprazan (Z) and naproxen (N), and the data is expressed as mean ± standard deviation.)

### D. Summary

No clinically significant PK interactions were observed between zastaprazan and aceclofenac, meloxicam, or naproxen, and there were no safety concerns.

### Example 3. Evaluation of the efficacy and safety of zastaprazan administration for preventing NSAID-induced peptic ulcer

### 1. Subject selection

A multicenter, parallel design, double-blind, randomized, active-controlled, non-inferiority clinical trial was designed to evaluate the efficacy and safety of zastaprazan for the prevention of gastroduodenal ulcer in patients with NSAID-induced peptic ulcer.

### Inclusion criteria

Eligible subjects must meet all of the following criteria:
1. Adult men and women aged 19 years or older as of the date of written consent
2. Those diagnosed with musculoskeletal diseases such as rheumatoid arthritis, osteoarthritis, and ankylosing spondylitis at the time of screening and requiring continuous administration of NSAIDs (aceclofenac, meloxicam, naproxen, celecoxib) for more than 24 weeks
3. Those with one or more of the following risk factors for ulcer development at the time of screening
   1) Age 60 years or older
   2) Those with a documented history of peptic ulcer confirmed by upper gastrointestinal endoscopy or those with ulcer scars confirmed by upper gastrointestinal endoscopy (performed within 10 days of randomization)
   3) Concomitant use of low-dose aspirin (aspirin <81 mg to 325 mg/day) (However, those with this risk factor must continue to take low-dose aspirin during the clinical trial period.)
   4) Concomitant use of prednisone at a dose of 10 mg/day or less (or corticosteroids of equivalent dose or lower) (However, those with this risk factor must maintain the administration of corticosteroids at the dose during the clinical trial period.)
4. Those who fully understand this clinical trial and voluntarily consent in writing to participate in the clinical trial

### Exclusion criteria

Eligible subjects must not meet any of the following criteria:
1. Those who are unable to undergo an endoscopy
2. Those who have an active ulcer (A1, A2) or a healing ulcer (H1, H2) in the stomach or duodenum according to the Sakita-Miwa classification* as a result of an upper gastrointestinal endoscopy at the time of screening

### * Sakita-Miwa classification

### 1) Active ulcer

(1) Active stage (A1): The white exudate is thick and protrudes around some areas. Small hemorrhages or clots often occur. The inflammatory symptoms such as surrounding redness, edema, and erosion are severe, and the raised margins are also large and high.
(2) Active stage (A2): The protruding white exudate disappears and its margins become clear. The surrounding inflammation also becomes considerably lighter, and the raised margins are slightly reduced. The mucosal rugae become concentrated. The regenerated epithelium is present, but very little.

### 2) Healing stage ulcer

(1) Healing stage (H1): The ulcer becomes shallow, the white exudate is clean, and the margin is smooth. The regenerated epithelium becomes clear, and the main body almost disappears, and the mucosal rugae reach the margins of the white exudate and are concentrated.
(2) Healing stage (H2): The ulcer shrinks significantly. The white exudate becomes thinner. The width of the regenerated epithelium becomes wider.

### 3) Scarring ulcers

(1) Scarring stage (S1): The mucosal loss disappears, but the erythema of the regenerated epithelium remains. Small discolored spots are often seen in the center. The mucosal rugae extend gently to the center.
(2) Scarring stage (S2): The erythema disappears, and the regenerated epithelium is thick and has a color very similar to the surrounding mucosa. The concentration of rugae also disappears, so only the concentration of the mucosa is seen in some cases.

### 3. Medical history

1) Those with a history of malignant tumor within five years from the time of screening. However, those with a history of malignant tumor of the digestive system are excluded regardless of the period.
2) Those with premonitory symptoms that may suggest a malignant disease of the gastrointestinal tract (dysphagia, severe dysphagia, bleeding, weight loss, anemia, bloody stool, etc.) (However, those who are found negative after confirming malignancy through an endoscopy and esophageal biopsy are excluded.)
3) Patients with gastroesophageal varices, Barrett's esophagus (greater than 3 cm), esophageal dysplasia, esophageal stricture, ulcerative stricture, or acute gastrointestinal bleeding as a result of screening upper gastrointestinal endoscopy
4) Those with or have a history of Zollinger-Ellison syndrome or other gastric acid secretion disorders
5) Those with or suspected of having inflammatory bowel disease (IBD), including pancreatitis, primary esophageal motility disorder, irritable bowel syndrome (IBS), Crohn's disease, ulcerative colitis, and Behcet's enteritis
6) Those who have undergone or are scheduled to undergo gastrointestinal or esophageal surgery such as gastric acid suppression surgery, gastrectomy, and endogastrectomy (excluding appendectomy, cholecystectomy, and polypectomy)
7) Those confirmed positive for *H. pylori* in a screening test
8) Those with clinically significant diseases of the blood, liver, kidneys, cardiovascular system, respiratory system, endocrine system, urinary system, neuropsychiatric system, immune system, blood tumor, and the like.
9) Those having a history of drug or alcohol abuse within one year from the screening date

### 4. Clinical laboratory tests

1) Those who meet any of the following criteria as a result of screening
   ① Alanine aminotransferase (ALT) or aspartate aminotransferase (AST) levels exceeding twice the upper limit of normal (ULN)
   ② Total bilirubin levels exceeding twice the ULN
   ③ Alkaline phosphatase (ALP) or γ-glutamyl Transpeptidase (γ-GTP) levels exceeding twice the ULN
2) Those who are confirmed positive in any of the following results: Human immunodeficiency virus (HIV) antigen/antibody test, hepatitis B surface antigen (HBsAg), hepatitis C antibody (anti-hepatitis C virus, Anti-HCV) test, or syphilis test (venereal disease research laboratory, VDRL)
   However, the following cases may be included:
   2-1) Those who are positive for HBsAg and have an hepatitis B virus (HBV)-DNA level of less than 100,000 IU/mL
   2-2) Those who are positive for hepatitis C virus (HCV)-Ab and negative for HCV-RNA
3) Those who are confirmed to have clinically significant (CS) abnormalities in an electrocardiography during screening

### 5. Allergy and drug hypersensitivity

1) Those known to have hypersensitivity to clinical trial drugs and components thereof
2) Those with a history of CS allergic disease (excluding mild allergic rhinitis that does not require administration) or hypersensitivity (asthma, acute rhinitis, nasal polyps, angioedema, urticaria, allergic reactions, etc.) to other drugs (NSAIDs, aspirin, antibiotics, etc.)

### 6. Contraindicated drugs and therapies

1) Those who must continuously take corticosteroids, antiplatelet agents, and anticoagulants during this clinical trial [However, the following cases are permitted.]
   ① Low-dose aspirin (81 mg or more to 325 mg/day) taken for the purpose of preventing cardiovascular disease before participating in this clinical trial
   ② Prednisone administration at 10 mg/day (or corticosteroids of an equivalent dose or less)
   ③ Topical corticosteroids used for the treatment of musculoskeletal diseases such as rheumatoid arthritis, osteoarthritis, and ankylosing spondylitis
2) Those who are taking or need to take drugs that are contraindicated for concomitant use in this clinical trial (However, those who have taken drugs that are contraindicated for concomitant use can participate after a one-week wash-out period. However, those who have taken drugs the following contraindicated for concomitant drugs may participate in this clinical trial after the corresponding wash-out period depending on the half-life of the drug.)

Drugs with a half-life exceeding 24 hours: Two weeks

When the period equivalent to 5 times the half-life of a drug exceed two weeks: A period five times the wash-out half-life of the drug

### 7. Pregnant or lactating women or those who have a positive pregnancy test result during screening

### 8. Contraception

1) Subjects and spouses (or partners) who do not use medically acceptable contraception during the clinical trial. The following methods are considered 'medically acceptable contraception':
   ① Use of an intrauterine device with a proven pregnancy failure rate
   ② Use of a double-barrier contraception method (male condom and closure cap, diaphragm, or cervical cap) and simultaneous use of spermicide
   ③ Sterilization (vasectomy, tubectomy and tubal ligation, hysterectomy)

### 9. Those with genetic problems such as galactose intolerance, Lapp lactase deficiency, or glucose-galactose malabsorption

### 10. Those who participated in another clinical trial within four weeks prior to participating in this clinical trial and were administered or applied any clinical trial drug or medical device at least once

### 11. Those who are deemed ineligible for participation in this clinical trial based on the medical opinion of the investigator

### 2. Administration dose, administration period, and administration method

The subjects are administered the clinical trial drug assigned to the randomly assigned administration group, one tablet and one capsule once a day before meals for 24 weeks from the day after the random assignment date, and are administered orally at a consistent time if possible.

**[Table 4]**

| Administration group | Oral administration once a day |
|---|---|
| Test group | ●Δ |
| Control group | ○▲ |

(In Table 4 above, • is JP-1366, 10 mg, o is JP-1366, 10 mg placebo, ▲ is lansoprazole, 15 mg, and Δ is lansoprazole, 15 mg placebo.)

### Co-administered NSAIDs

The subjects will be administered one type of NSAIDs among aceclofenac, meloxicam, naproxen, and celecoxib at the investigator's discretion from the date of randomization, and the type and dose of the co-administered NSAID must be maintained without change during the clinical trial period.

### 3. Clinical trial method

Subjects visit the clinical trial institution at week 4, 12, and 24 from the randomization date to conduct efficacy and safety evaluations according to the designated schedule. The efficacy evaluation includes confirming the occurrence of peptic ulcer and gastrointestinal symptoms associated with NSAIDs.

The safety evaluation includes collecting information on adverse events (AEs), vital signs (blood pressure, pulse, body temperature), physical examinations, and clinical laboratory tests (hematology, blood chemistry, and urine tests).

When peptic ulcer is confirmed by an endoscopy during the clinical trial period, clinical participation is terminated early at that point. When an ulcer is confirmed and treatment is terminated early, it is considered as end of treatment (EOT). A follow-up (F/U) visit is conducted after EOT.

### 4. Efficacy endpoints

### A. Primary efficacy endpoint:

Cumulative proportion (%) of subjects with peptic ulcer* up to week 24
*Active stage/healing stage ulcer according to Sakita-Miwa classification or newly discovered scarring stage ulcer other than scars identified at screening

### B. Secondary efficacy endpoint:

Proportion (%) of subjects with endoscopic bleeding** of the stomach or duodenum up to week 24
**Those corresponding to Class 1 or 2 according to Forrest classification

### C. Exploratory endpoints

1. Proportion (%) of subjects with peptic ulcer up to week 12
2. Proportion (%) of subjects with endoscopic bleeding** of the stomach or duodenum up to week 12
3. Peptic ulcer incidence rate (%) by type of NSAIDs (aceclofenac, meloxicam, naproxen, celecoxib)
4. Peptic ulcer incidence rate by peptic ulcer onset risk factor items
5. Peptic ulcer incidence rate by the number of peptic ulcer onset risk factors
6. Change in serum gastrin concentration compared to baseline
7. Proportion (%) of gastrointestinal symptoms through questionnaire (00) compared to baseline at weeks 4, 12, and 24
8. Change in blood hemoglobin (Hb) concentration compared to baseline at weeks 4, 12, and 24

### 5. Safety endpoints

- Adverse events
- Vital signs
- Physical examination
- Clinical laboratory test results
- Electrocardiogram

### 6. Definition of analysis groups and statistical analysis method

### Definition of analysis groups

The analysis group is divided into the following three groups, and efficacy evaluation analysis is performed based on full analysis set (FAS) and per protocol set (PPS), with FAS as the main analysis group. A safety analysis is performed on the safety set (safety assessment group).

### 1. FAS

This refers to the group of subjects who were administered the clinical trial drug at least once and underwent the primary efficacy assessment at least once in this clinical trial.

### 2. PPS

This refers to all subjects included in the FAS who did not have any serious violations of the clinical trial protocol.

### 3. Safety set (safety assessment group)

This refers to the group of subjects who were administered the clinical trial drug at least once in this clinical trial.

### Statistical analysis method

### A. Primary efficacy endpoint

Cumulative proportion (%) of subjects with peptic ulcer up to week 24
: The proportion of subjects with peptic ulcer up to week 24 after drug administration compared to baseline is presented by treatment group, and to compare whether there is a difference between treatment groups, when cells with an expected frequency of less than 5 are less than 20% of the total, a Chi-square test is performed, and when cells with an expected frequency of less than 5 are more than or equal to 20% of the total, a Fisher's exact test is performed.

In addition, a logistic regression analysis is performed to confirm whether there are differences between treatment groups when the occurrence of peptic ulcer at week 24 is adjusted for the type of NSAIDs (aceclofenac, meloxicam, naproxen, celecoxib) being administered to the stratified factor subjects (e.g., peptic ulcer occurrence at 24 weeks) and the administration (used/unused) of aspirin or/and prednisone among the risk factors of ulcer occurrence.

### B. Secondary efficacy endpoint

Proportion (%) of subjects with endoscopic bleeding of the stomach or duodenum up to week 24
:The proportion of subjects with endoscopic bleeding of the stomach or duodenum up to week 12 and week 24 after drug administration compared to baseline is presented by treatment group, and to compare whether there is a difference between treatment groups, when cells with an expected frequency of less than 5 are less than 20% of the total, a Chi-square test is performed, and when cells with an expected frequency of less than 5 are more than or equal to 20% of the total, a Fisher's exact test is performed.

### C. Exploratory endpoints

1. Proportion (%) of subjects with peptic ulcers up to 12 weeks
   : The proportion of subjects with peptic ulcer up to week 12 after drug administration compared to baseline is presented by treatment group, and to compare whether there is a difference between treatment groups, when cells with an expected frequency of less than 5 are less than 20% of the total, a Chi-square test is performed, and when cells with an expected frequency of less than 5 are more than or equal to 20% of the total, a Fisher's exact test is performed.
2. Proportion (%) of subjects with endoscopic bleeding** of the stomach or duodenum up to week 12
   :The proportion of subjects with endoscopic bleeding in the stomach or duodenum at weeks 12 and 24 after drug administration compared to the baseline is presented by treatment group, and to compare whether there is a difference between treatment groups, when cells with an expected frequency of less than 5 are less than 20% of the total, a Chi-square test is performed, and when cells with an expected frequency of less than 5 are more than or equal to 20% of the total, a Fisher's exact test is performed.
3. Peptic ulcer incidence rate (%) by type of NSAIDs (aceclofenac, meloxicam, naproxen, celecoxib)
4. Peptic ulcer incidence rate (%) by peptic ulcer onset risk factor items
   :The peptic ulcer incidence rate by peptic ulcer onset risk factor items is presented by treatment group, and to compare whether there is a difference between treatment groups, when cells with an expected frequency of less than 5 are less than 20% of the total, a Chi-square test is performed, and when cells with an expected frequency of less than 5 are more than or equal to 20% of the total, a Fisher's exact test is performed.
5. Peptic ulcer incidence rate (%) by the number of peptic ulcer onset risk factors
6. Change in gastrin levels compared to baseline at weeks 4, 12, and 24
   Descriptive statistics (mean, standard deviation, median, minimum, maximum) are described for gastrin levels at weeks 4, 12, and 24 and for changes in gastrin levels from baseline up to weeks 4, 12, and 24 after drug administration, and to compare whether there is a difference between treatment groups, when the normality assumption is satisfied, a two-sample t-test is performed, and when the normality assumption is not satisfied, a Wilcoxon rank sum test is performed. In addition, when the changes in the test value at each visit compared to the baseline are not compared, a Wilcoxon's signed rank test is used for analysis.
7. Proportion (%) of gastrointestinal symptoms through questionnaire compared to baseline at weeks 4, 12, and 24
   The proportion of subjects without NSAIDs-related gastrointestinal symptoms (such as acid reflux, upper abdominal pain or discomfort, or indigestion) at weeks 4, 12, and 24 after drug administration is presented by treatment group, and to compare whether there is a difference between treatment groups, when cells with an expected frequency of less than 5 are less than 20% of the total, a Chi-square test is performed, and when cells with an expected frequency of less than 5 are more than or equal to 20% of the total, a Fisher's exact test is performed.
8. Change (%) in blood hemoglobin (Hb) concentration compared to baseline at weeks 4, 12, and 24

Descriptive statistics (mean, standard deviation, median, minimum, maximum) are described for the blood hemoglobin levels at weeks 4, 12, and 24 and the changes in hemoglobin levels from baseline up to weeks 4, 12, and 24 after drug administration, and to compare whether there is a difference between treatment groups, when the normality assumption is satisfied, a two-sample t-test is performed, and when the normality assumption is not satisfied, a Wilcoxon rank sum test is performed. In addition, when the changes in the test value at each visit compared to the baseline are not compared, a Wilcoxon's signed rank test is used for analysis.
*When medication compliance is less than or equal to 80%, it is considered investigational product (IP) non-compliance.

### D. Safety endpoints

### 1. Adverse reactions

The number and percentage of subjects with adverse reactions, 95% confidence interval, and the number of occurrences are presented by treatment group, and to compare whether there is a difference between treatment groups, when cells with an expected frequency of less than 5 are less than 20% of the total, a Chi-square test is performed, and when cells with an expected frequency of less than 5 are more than or equal to 20% of the total, a Fisher's exact test is performed.

The number and percentage of subjects with adverse reactions, 95% confidence interval, and the number of occurrences are presented by treatment group for the severity of adverse reactions, causality, actions for clinical trial drugs, and treatment of adverse events and their results, and to compare whether there is a difference between treatment groups, when cells with an expected frequency of less than 5 are 20% or less of the total, a Chi-square test is performed, and when cells with an expected frequency of less than 5 are more than 20% of the total, a Fisher's exact test is performed.

### 2. Vital signs, physical examination, and clinical laboratory tests

For continuous data, descriptive statistics (mean, standard deviation, median, minimum, maximum) are described for each visit, and to compare whether there is a difference between treatment groups, when the normality assumption is satisfied, a two-sample t-test is performed, and when the normality assumption is not satisfied, a Wilcoxon rank sum test is performed. In addition, when the changes in the test value at each visit compared to the baseline are not compared, a Wilcoxon's signed rank test is used for analysis.

For categorical data, the frequency and proportion for each visit are described, and to compare whether there is a difference between treatment groups, when cells with an expected frequency of less than 5 are less than 20% of the total, a Chi-square test is performed, and when cells with an expected frequency of less than 5 are more than or equal to 20% of the total, a Fisher's exact test is performed. The values at baseline and at each visit are divided into normal/abnormal categories and a McNemar's Test is performed to test whether the normal/abnormal ratio changed before and after administration of the clinical trial drug.

While specific parts of the present invention have been described in detail above, it is obvious to those skilled in the art that these specific descriptions are merely preferred embodiments and that the scope of the present invention is not limited thereto. Accordingly, the actual scope of the present invention will be defined by the appended claims and their equivalents.

## Claims

1. A pharmaceutical composition comprising zastaprazan, a pharmaceutically acceptable salt thereof, a hydrate or solvate thereof, or a mixture thereof for preventing, treating, or preventing recurrence of peptic ulcer, wherein the peptic ulcer is caused by administration of nonsteroidal anti-inflammatory drugs (NSAIDs).

2. The pharmaceutical composition according to claim 1, wherein the pharmaceutically acceptable salt of zastaprazan is a zastaprazan citrate salt.

3. The pharmaceutical composition according to claim 1, wherein the pharmaceutical composition comprises zastaprazan, a pharmaceutically acceptable salt thereof, a hydrate or solvate thereof, or a mixture thereof in an amount of 1 mg to 100 mg as zastaprazan.

4. The pharmaceutical composition according to claim 1, wherein the pharmaceutical composition comprises a zastaprazan citrate salt in an amount of 5 to 40 mg.

5. The pharmaceutical composition according to claim 1, wherein the pharmaceutical composition comprises a zastaprazan citrate salt in an amount of 10 mg.

6. The pharmaceutical composition according to claim 1, wherein a subject administered with the pharmaceutical composition is a subject who has experienced peptic ulcer caused by administration of NSAIDs.

7. The pharmaceutical composition according to claim 1, wherein a subject administered with the pharmaceutical composition is a subject who has experienced curing of peptic ulcer.

8. The pharmaceutical composition according to claim 1, wherein the NSAIDs are acetic acid-derived non-selective NSAIDs, enolic acid-derived non-selective NSAIDs, fenamic acid-derived non-selective NSAIDs, p-amino phenol-derived non-selective NSAIDs, propionic acid-derived non-selective NSAIDs, salicylic acid-derived non-selective NSAIDs, selective COX-2 inhibitors, or mixtures thereof.

9. The pharmaceutical composition according to claim 8, wherein the acetic acid-derived non-selective NSAIDs are aceclofenac, acemetacin, actarit, alcofenac, amfenac, clometacin, diclofenac, etodolac, felbinac, fenclofenac, indometacin, ketorolac, metiazinic acid, mofezolac, nabumetone, naproxen, oxametacin, sulindac, zomepirac, or any combination thereof;
wherein the enolic acid-derived NSAIDs are droxicam, isoxicam, lornoxicam, meloxicam, piroxicam, tenoxicam, or any combination thereof;
wherein the fenamic acid-derived NSAIDs derived are flufenamic acid, mefenamic acid, meclofenamic acid, tolfenamic acid, or any combination thereof;
wherein the p-amino phenol-derived NSAIDs are paracetamol, phenacetin, or any combination thereof;
wherein the propionic acid-derived NSAIDs are alminoprofen, benoxaprofen, dexketoprofen, fenoprofen, flurbiprofen, ibuprofen, indoprofen, ketoprofen in a pharmaceutically acceptable form, loxoprofen, oxaprozin, pranoprofen, suprofen, or any combination thereof;
wherein the salicylic acid-derived NSAIDs are acetylsalicylic acid, diflunisal, salsalate, or any combination thereof; and
wherein the selective COX-2 inhibitors are celecoxib, rofecoxib, valdecoxib, parecoxib, lumiracoxib, etoricoxib, firocoxib, or any combination thereof.

10. The pharmaceutical composition according to claim 1, wherein the NSAIDs are one or more selected from the group consisting of naproxen, aceclofenac, meloxicam, and celecoxib.

11. The pharmaceutical composition according to claim 1, wherein the pharmaceutical composition is administered once a day.

12. The pharmaceutical composition according to claim 1, wherein the pharmaceutical composition is formulated into a solid oral preparation.

13. A use of a pharmaceutical composition for preventing peptic ulcer induced by administration of nonsteroidal anti-inflammatory drugs (NSAIDs), the pharmaceutical composition comprising zastaprazan, a pharmaceutically acceptable salt thereof, a hydrate or solvate thereof, or a mixture thereof.

14. A use of a pharmaceutical composition for preventing recurrence of peptic ulcer induced by administration of NSAIDs, the pharmaceutical composition comprising zastaprazan, a pharmaceutically acceptable salt thereof, a hydrate or solvate thereof, or a mixture thereof.

15. A use a pharmaceutical composition for preparing a medicament for preventing peptic ulcer induced by administration of NSAIDs, the pharmaceutical composition comprising zastaprazan, a pharmaceutically acceptable salt thereof, a hydrate or solvate thereof, or a mixture thereof.

16. A use a pharmaceutical composition for preparing a medicament for preventing recurrence of peptic ulcer induced by administration of NSAIDs, the pharmaceutical composition comprising zastaprazan, a pharmaceutically acceptable salt thereof, a hydrate or solvate thereof, or a mixture thereof.

17. A method of preventing recurrence or onset of peptic ulcer induced by administration of NSAIDs, by administering to a subject a pharmaceutical composition comprising zastaprazan, a pharmaceutically acceptable salt thereof, a hydrate or solvate thereof, or a mixture thereof, in a pharmaceutically effective amount.
